# EUROPEAN PATENT SPECIFICATION

(11) **EP 0 439 353 B1**
(45) Date of publication and mention of the grant of the patent: **24.04.1996**
(21) Application number: 91300539.3
(22) Date of filing: 24.01.1991
(51) Int. Cl.: C07D 463/00, A61K 31/435

(54) **Crystalline hydrochloride of new beta-lactam antibiotic and process therefor**
Kristallines Hydrochlorid eines neuen beta-Lactam-Antibiotikums und Verfahren zur Herstellung
Hydrochlorure cristallin d'un nouvel antibiotique bêta-lactame et procédé de préparation

(30) Priority: 26.01.1990 US 470486
(43) Date of publication of application: 31.07.1991
(73) Proprietor: ELI LILLY AND COMPANY, Indianapolis, Indiana 46285 (US)
(72) Inventor: Pfeiffer, Ralph Robert, Indianapolis, Indiana 46220 (US)
(74) Representative: Tapping, Kenneth George

(56) References cited:
- EP-A- 0 014 476
- EP-A- 0 311 366
- EP-A- 0 369 686
- EP-A- 0 369 687

## Description

The β-lactam antibiotic of the formula 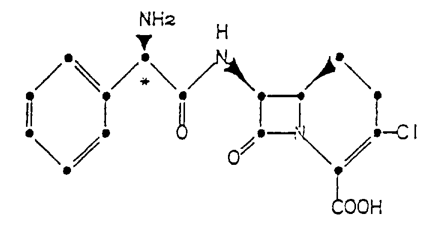 is a potent new orally-active antibiotic. The antibiotic is described, for example, by J. Hashimoto et al. in U.S. Patent No. 4,335,211, issued June 15, 1982, herein incorporated by reference. The compound of the above formula will be referred to herein by the designation LY163892.

The instant invention is directed to the crystalline hydrochloride of LY163892. This compound is a pharmaceutically elegant form of the parent compound. In addition, LY163892 crystalline hydrochloride is a convenient intermediate for the purification of LY163892 crystalline monohydrate. LY163892 crystalline monohydrate is a pharmaceutically elegant form of the parent compound possessing excellent stability and bulk handling qualities over a wide range of humidity and other manufacturing conditions. LY163892 crystalline monohydrate is further described by C. Pasini in European Patent Application No. 311,366, published April 12, 1989, entitled MONOHYDRATE OF NEW BETA-LACTAM ANTIBIOTIC.

One aspect of the present invention is directed to the crystalline hydrochloride of LY163892. More specifically, the invention is directed to LY163892 crystalline hydrochloride having the X-ray powder diffraction pattern listed below in Table I. Other aspects of the invention are pharmaceutical formulations of LY163892 crystalline hydrochloride. Yet another aspect of the instant invention is a process for preparing LY163892 crystalline hydrochloride which comprises acidifying LY163892 in acetonitrile in the presence of a source of chloride ions.

The instant invention is directed to the crystalline hydrochloride of LY163892, the antibiotic compound of Formula I:

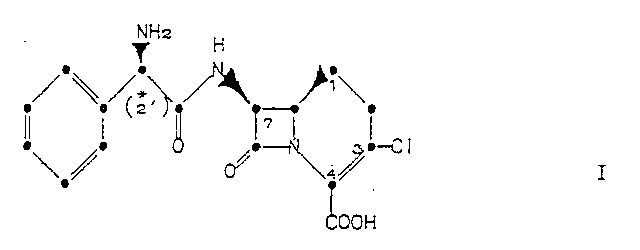

The 2′ position carbon of the antibiotic in Formula I exists as the "R" absolute configuration. Furthermore, the compound of Formula I can also be in the form of a zwitterion, a form which is also encompassed in the various aspects of the instant invention. The compound of the instant invention will be referred to herein as either "LY163892 crystalline hydrochloride" or, more simply, "the crystalline hydrochloride".

The crystalline hydrochloride is a white crystalline solid. A preferred embodiment of the invention is the crystalline hydrochloride characterized by the X-ray powder diffraction pattern of Table I:

**Table I**

| Crystalline Hydrochloride Diffraction Pattern | |
|---|---|
| d | I/I₁ |
| 13.57 | 0.56 |
| 9.30 | .07 |
| 8.99 | .10 |
| 7.37 | .18 |
| 6.89 | .37 |
| | |
| 5.86 | .12 |
| 5.56 | .53 |
| 5.26 | .10 |
| 4.83 | .07 |
| 4.32 | .12 |
| | |
| 4.00 | .10 |
| 3.77 | .64 |
| 3.68 | 1.00 |
| 3.60 | .58 |
| 3.47 | .92 |
| 3.13 | .25 |

The diffraction pattern in Table I was obtained with nickel-filtered copper radiation of wavelength λ 1.5405 Å. A scan range of 4.0 to 35.0 degrees two theta was employed. The interplanar spacings are in the column marked "d" and the relative intensities are in the column marked "I/I₁".

Further aspects of the present invention are pharmaceutical formulations of LY163892 crystalline hydrochloride. Preferred embodiments of the invention include pharmaceutical formulations containing the crystalline hydrochloride form of LY163892 characterized by the diffraction pattern of Table I.

Yet another embodiment of the present invention is a process for preparing LY163892 crystalline hydrochloride. The crystalline hydrochloride is prepared in acetonitrile from any of the various forms of the parent compound, including the anhydrate, hydrates, ethanol solvate, the various DMF solvates, or the various combinations of DMF solvate - hydrate. The preferred starting material for the crystalline hydrochloride is the anhydrate.

According to the present process and acid addition salt of LY163892 is formed in acetonitrile in the presence of a source of chloride ions. LY163892 crystalline hydrochloride precipitates from the mixture.

In dilute suspensions, the starting material may dissolve completely to form a clear solution before the crystalline hydrochloride begins to precipitate. In more concentrated suspensions, the same phenomena occurs locally even though it may appear that the bulk starting material does not dissolve completely.

Preferably, concentrated hydrochloric acid is added to a solution/suspension of LY163892 in acetonitrile to form the crystalline hydrochloride. Alternatively, an acid addition salt of LY163892 can be formed by adding another acid, such as nitric, sulfuric or phosphoric acid, to a solution/suspension of LY163892 or a solvate thereof in acetonitrile, and thereafter the crystalline hydrochloride salt can be formed and precipitated from the acidified mixture by the addition of a source of chloride ions. The source of chloride ions, preferably an alkali metal chloride or other water soluble chloride or hydrochloride salt, can be conveniently introduced into the acidified acetonitrile solution, for example, in aqueous solution. Alternatively, the source of chloride ions can be added to the mixture either before or with the acid addition. Acetonitrile, or more precisely, acetonitrile containing about 1 to about 5 percent water (from the added acid), has been the only solvent found operable for the generation and precipitation of the present crystalline hydrochloride. No LY163897 crystalline hydrochloride precipitate has been obtained using other water misible solvents.

The crystalline hydrochloride of the present invention may itself be used, after drying to remove residual solvent, in pharmaceutical formulations, or it may be readily converted to the pharmaceutically elegant monohydrate form of LY163892. Crystallization of the desired monohydrate form of LY163892 is induced by slowly adjusting the pH of an aqueous solution of the present crystalline hydrochloride with base to about 2 to about 6, preferably about 4.8. Any of a variety of bases may be employed such as the alkali metal hydroxides, for example sodium hydroxide and potassium hydroxide, the organic bases such as triethylamine and diisopropylamine, or related bases such as ammonium hydroxide. The product monohydrate is isolated by filtration of the resulting suspension using conventional filtering techniques, such as vacuum filtration on a Büchner funnel. The collected crystals are washed and allowed to dry in air at ambient temperature. Alternatively, warm pH-adjusted suspensions (50°C) are cooled to approximately 20°C, stirred, filtered, and the collected monohydrate is dried at 30°C for 24 to 48 hours.

As stated above, in a further aspect of this invention there are provided pharmaceutical compositions of LY163892 crystalline hydrochloride. Such pharmaceutical compositions are useful for the control of gram-positive and gram-negative bacterial infections in warm-blooded animals and comprise a suitable vehicle and a therapeutically effective amount of the present crystalline hydrochloride.

With regard to compositions for oral administration such as tablets and capsules, the term "suitable vehicle" means common excipients such as binding agents, for example, syrup, acacia, gelatin, sorbitol, tragacanth, polyvinylpyrrolidine (Povidone), methylcellulose, ethylcellulose, sodium carboxymethylcellulose, hydroxypropylmethylcellulose, sucrose, and starch; fillers and carriers, for example corn starch, gelatin, lactose, sucrose, microcrystalline cellulose, kaolin, mannitol, dicalcium phosphate, sodium chloride, and alginic acid; disintegrators such as croscarmellose sodium, microcrystalline cellulose, corn starch, sodium starch glycolate, alginic acid; suitable wetting agents such as sodium lauryl sulfate; and lubricants such as magnesium stearate and other metallic stearates, stearic acid, silicone fluid, talc, waxes, oils and colloidal silica. Flavoring agents such as peppermint, oil of wintergreen, cherry flavoring or the like can also be used. It may be desirable to add a coloring agent to make the dosage form more aesthetically pleasing or to help identify the product. The tablets may also be coated by methods well known in the art.

The pharmaceutical compositions of the present invention may also be in the form of oral liquid preparations, which may be either a) suspensions, solutions, emulsions or syrups, or b) a dry powder to be reconstituted with water or another suitable vehicle before use. When used in conjunction with such oral liquid preparations, the term "suitable vehicle" means conventional additives such as suspending agents such as acacia, methyl cellulose, hydroxyethyl cellulose, carboxymethyl cellulose, cellulose with sodium carboxymethyl cellulose (Avicel®), xantham gum, or starch; sweeteners such as sucrose, syrup, glucose, saccharin, sorbital, or aspartame; wetting agents such as sodium lauryl sulfate, silicone oil, the various Pluronics® surfactants, or glycerin; preservatives such as methyl, propyl, or butyl p-hydroxybenzoates, or sorbic acid; dyes, flavors and salts such as sodium chloride, citric acid, oil of wintergreen or sodium citrate; and oils and esters such as almond oil, fractionated coconut oil, hydrogenated castor oil, lecithin, aluminum stearate, and the like.

Topical compositions can be formulated with "suitable vehicles" such as hydrophobic or hydrophilic bases. Such bases include ointments, creams or lotions.

Veterinary pharmaceutical compositions of the antibiotic compound may be administered in the feed or the drinking water of farm animals. Alternatively, the compounds can be formulated as intramammary preparations with "suitable vehicles" such as long- or quick-release bases.

A "therapeutically effective amount" of the instant crystalline hydrochloride is from approximately 2.5 mg to about 70 mg of compound per kilogram of body weight per dose. This amount generally totals from about 200 milligrams to about 7 grams per day for an adult human.

During a course of treatment with the instant crystalline hydrochloride, the crystalline hydrochloride can be administered in a single daily dose or in multiple doses per day. The treatment regime may require administration over extended periods of time, for example, for several days or for from two to three weeks. The amount administered per dose or the total amount administered will depend on such factors as the nature and severity of the infection, the age and general health of the patient, and the tolerance of both the patient and the microorganism or microorganisms involved in the infection to the crystalline hydrochloride. The following Examples further illustrate specific aspects of the present invention. The Examples are not intended to be limiting to the scope of the invention in any respect and should not be so construed.

### Example 1

### LY163892 Crystalline Hydrochloride

Four hundred milligrams of LY163892 anhydrate were suspended in 10 ml of acetonitrile. To the suspension was added sufficient concentrated hydrochloric acid to convert the suspended anhydrate to the hydrochloride salt. The precipitated LY163892 crystalline hydrochloride was collected by vacuum filtration. The powder diffraction X-ray parameters and pattern for this compound are given above in Table 1.

### Example 2

### LY163892 Crystalline Hydrochloride

Concentrated hydrochloric acid (1.5 ml) was added to a suspension of 5.0 g of LY163092 anhydrate in 50 ml of acetonitrile. The precipitated crystals were collected by vacuum filtration and washed with 10 ml of acetonitrile to provide 4.7 g of LY163892 crystalline hydrochloride following air drying overnight.

The crystalline hydrochloride was converted to LY163892 monohydrate as follows. LY163892 crystalline hydrochloride (4.7 g) was combined with 35 ml of water to provide a solution having a pH of 1.6. The pH of the solution was raised to about 4 by the addition of ammonium hydroxide. Plates of LY163892 monohydrate formed in the solution and were isolated by vacuum filtration. The product was air dried. An X-ray diffraction pattern was generated and compared to an authentic sample of LY163892 monohydrate to confirm the identity of the monohydrate.

### Example 3

### LY163892 Crystalline Hydrochloride

To a suspension of 400 mg of LY163892 DMF solvate in 10 ml of acetonitrile is added 0.25 ml of concentrated nitric acid. The crystalline hydrochloride is formed and precipitates as 0.2 ml of a saturated sodium chloride solution is added to the solution. The crystalline hydrochloride is collected by vacuum filtration.

### Example 4

A dosage form in hard gelatin capsules is prepared using the following ingredients

| | Quantity (mg/capsule) |
|---|---|
| LY163892 crystalline hydrochloride | 200 |
| starch flowable powder | 186 |
| starch flowable powder with silicone 5% | 50 |
| magnesium stearate | 2.5 |

The above ingredients are mixed and filled into hard gelatin capsules in 438.5 mg quantities.

### Example 5

A tablet formula is prepared using the ingredients below:

| | Quantity (mg/tablet) |
|---|---|
| LY163892 crystalline hydrochloride | 200 |
| cellulose, microcrystalline | 200 |
| silicon dioxide, fumed | 10 |
| stearic acid | 5 |

The components are blended and compressed to form tablets each weighing 415 mg.

### Example 6

Suppositories each containing 200 mg of active ingredient are made as follows:

| | |
|---|---|
| LY163892 crystalline hydrochloride | 200 mg |
| saturated fatty acid glycerides to | 2000 mg |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

## Claims (Claims for the following Contracting State(s): DE, FR, GB, IT)

1. A crystalline hydrochloride of the compound of the formula

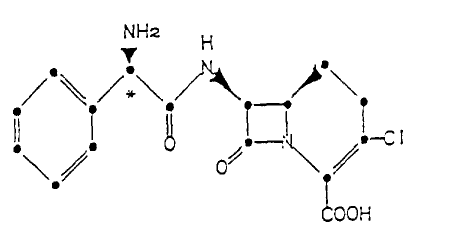

2. The compound of Claim 1 having the following X-ray powder diffraction pattern obtained with a nickel-filtered copper radiation of λ = 1.5405 Å wherein d represents the interplanar spacing and I/I₁ the relative intensity:
| d | I/I₁ |
|---|---|
| 13.57 | 0.56 |
| 9.30 | .07 |
| 8.99 | .10 |
| 7.37 | .18 |
| 6.89 | .37 |
| | |
| 5.86 | .12 |
| 5.56 | .53 |
| 5.26 | .10 |
| 4.83 | .07 |
| 4.32 | .12 |
| | |
| 4.00 | .10 |
| 3.77 | .64 |
| 3.68 | 1.00 |
| 3.60 | .58 |
| 3.47 | .92 |
| 3.13 | .25 |

3. A pharmaceutical formulation comprising as an active ingredient a compound as claimed in Claim 1 or 2, associated with one or more pharmaceutically acceptable carriers, excipients or diluents therefor.

4. A process for preparing the compound of Claim 1 or 2 which comprises the step of forming an acid addition salt of a compound of the formula 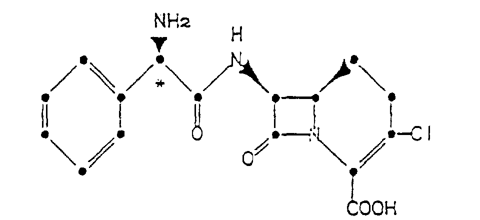 in acetonitrile in the presence of a source of chloride ions.

5. The process of Claim 4 wherein the acid addition salt is formed by the addition of hydrochloric acid.

6. A crystalline hydrochloride as claimed in Claim 1 or 2 for use as an anti-bacterial.

## Claims (Claims for the following Contracting State(s): ES)

1. A process for preparing a crystalline hydrochloride of a compound of the formula 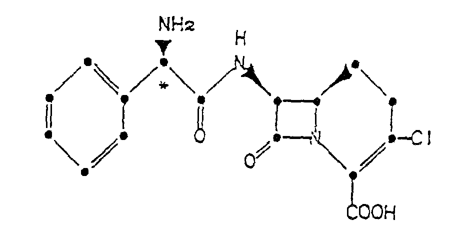 which comprises forming an acid addition salt of such compound in acetonitrile and in the presence of a source of chloride ions.

2. The process of Claim 1 wherein the acid addition salt is formed by the addition of hydrochloric acid.

3. A process for preparing a pharmaceutical formulation which comprises admixing a crystalline hydrochloride of the compound of the formula 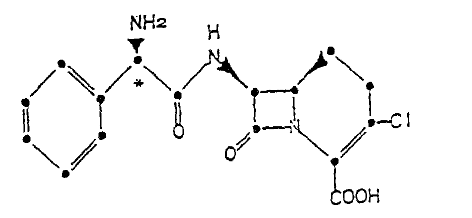 with one or more pharmaceutically-acceptable carriers, diluents, or excipients therefor.

## Patentansprüche (Patentansprüche für folgende(n) Vertragsstaat(en): DE, FR, GB, IT)

1. Kristallines Hydrochlorid der Verbindung der Formel

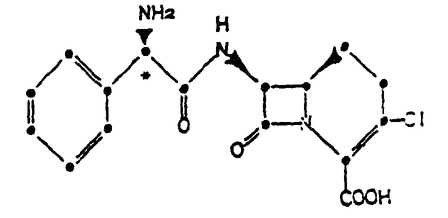

2. Verbindung der Formel 1 mit folgendem Röntgenbeugungsmuster am Pulver, das mit einer Nickel-gefilterten Kupferstrahlung mit λ = 0,15405 nm erhalten wird, worin d für den Interplanarabstand und I/I₁ für die relative Intensität stehen:
| d | I/I₁ |
|---|---|
| 13,57 | 0,56 |
| 9,30 | 0,07 |
| 8,99 | 0,10 |
| 7,37 | 0,18 |
| 6,89 | 0,37 |
| 5,86 | 0,12 |
| 5,56 | 0,53 |
| 5,26 | 0,10 |
| 4,83 | 0,07 |
| 4,32 | 0,12 |
| 4,00 | 0,10 |
| 3,77 | 0,64 |
| 3,68 | 1,00 |
| 3,60 | 0,58 |
| 3,47 | 0,92 |
| 3,13 | 0,25 |

3. Pharmazeutische Formulierung, die als Wirkstoff eine Verbindung nach Anspruch 1 oder 2 zusammen mit einem oder mehreren pharmazeutisch annehmbaren Trägern, Hilfstoffen oder Verdünnungsmitteln hierfür enthält.

4. Verfahren zur Herstellung der Verbindung nach Anspruch 1 oder 2, gekennzeichnet durch die Bildung eines Säureadditionssalzes einer Verbindung der Formel 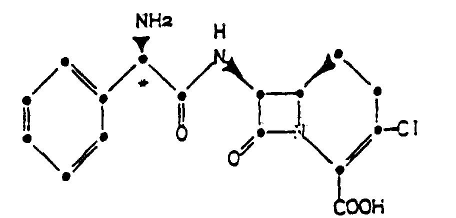 in Acetonitril in Gegenwart einer Chloridionenquelle.

5. Verfahren nach Anspruch 4, worin das Säureadditionssalz durch die Zugabe von Chlorwasserstoffsäure gebildet wird.

6. Kristallines Hydrochlorid nach Anspruch 1 oder 2 zur Verwendung als antibakterielles Mittel.

## Patentansprüche (Patentansprüche für folgende(n) Vertragsstaat(en): ES)

1. Verfahren zur Herstellung eines kristallinen Hydrochlorids einer Verbindung der Formel 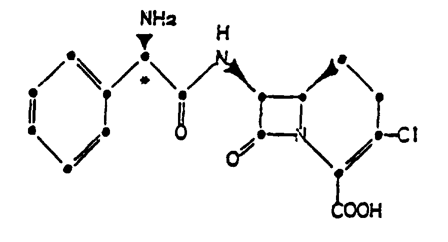 gekennzeichnet durch die Bildung eines Säureadditionssalzes einer solchen Verbindung in Acetonitril und in Gegenwart einer Chloridionenquelle.

2. Verfahren nach Anspruch 1, worin das Säureadditionssalz durch die Zugabe von Chlorwasserstoffsäure gebildet wird.

3. Verfahren zur Herstellung einer pharmazeutischen Formulierung, gekennzeichnet durch Mischen eines kristallinen Hydrochlorids einer Verbindung der Formel 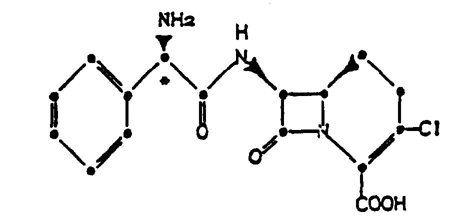 mit einem oder mehreren pharmazeutisch annehmbaren Trägern, Verdünnungsmitteln oder Hilfsstoffen hierfür.

## Revendications (Revendications pour l'(les) Etat(s) contractant(s) suivant(s): DE, FR, GB, IT)

1. Chlorhydrate cristallin du composé de formule :

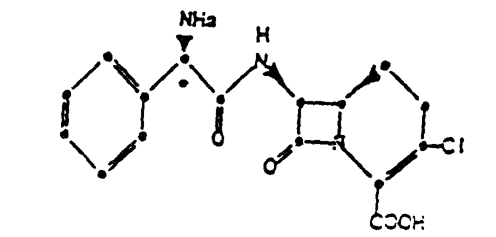

2. Composé suivant la revendication 1, ayant le schéma suivant de diffraction des rayons X de la poudre, obtenu au moyen d'un rayonnement de cuivre filtré par du nickel de λ = 1,5405 Å, dans lequel d représente la distance interplanaire et I/I₁, l'intensité relative :

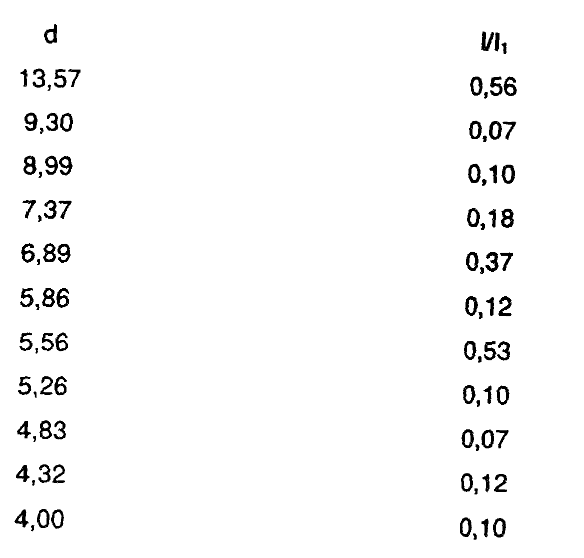

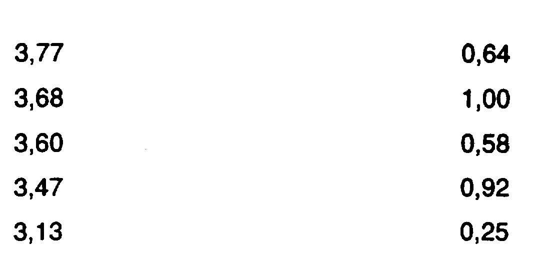

3. Formulation pharmaceutique comprenant comme ingrédient actif, un composé suivant la revendication ou 2, associé à un ou plusieurs supports, excipients ou diluants pharmaceutiquement acceptables.

4. Procédé de préparation du composé suivant la revendication 1 ou 2, qui comprend l'étape de formation d'un sel d'addition d'acide d'un composé de formule : 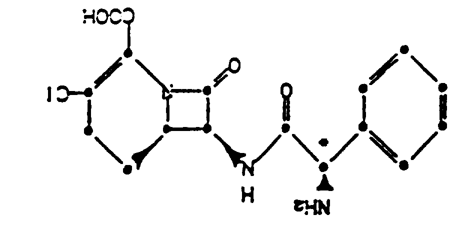 dans l'acétonitrile, en présence d'une source d'ions chlorure.

5. Procédé suivant la revendication 4, dans lequel le sel d'addition d'acide est formé par addition d'acide chlorhydrique.

6. Chlorhydrate cristallin suivant la revendication 1 ou 2 à utiliser comme agent antibactérien.

## Revendications (Revendications pour l'(les) Etat(s) contractant(s) suivant(s): ES)

1. Procédé de préparation d'un chlorhydrate cristallin d'un composé de formule: 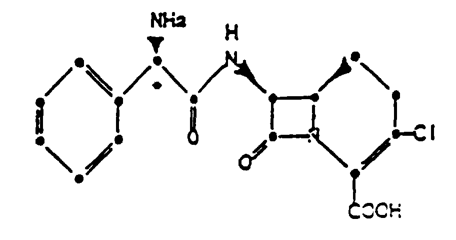 qui comprend la formation d'un sel d'addition d'acide du composé dans l'acétonitrile et en présence d'une source d'ions chlorure.

2. Procédé suivant la revendication 1, dans lequel le sel d'addition d'acide est formé par addition d'acide chlorhydrique.

3. Procédé de préparation d'une formulation pharmaceutique, qui comprend le mélange d'un chlorhydrate cristallin du composé de formule : 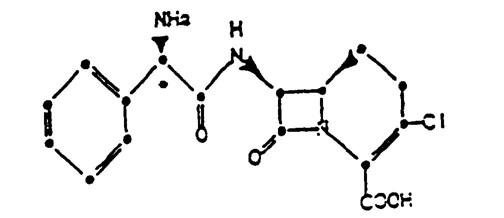 avec un ou plusieurs supports, diluants ou excipients pharmaceutiquement acceptables.
